# EUROPEAN PATENT APPLICATION

(11) **EP 4 000 645 A1**
(43) Date of publication of application: **25.05.2022**
(21) Application number: 20843061.1
(22) Date of filing: 17.07.2020
(51) Int. Cl.: A61L 2/10, A23L 3/28

(54) **ELECTRICAL HOUSEHOLD APPLIANCE AND ADDITIONAL ELECTROPASTEURIZER AND ELECTROSTERILIZER MODULE FOR FOOD AND UTENSILS, THROUGH THE HARMONIC SYNCHRONICITY OF ELECTRIC FIELDS, MAGNETIC FIELDS AND ELECTROMAGNETIC WAVE CONDENSING SYSTEM**

(30) Priority: 19.07.2019 BR 102019014977
(71) Applicant: Duvoisin, Charles Adriano, 88330-155 Balneário Camboriu, SC (BR)
(72) Inventor: BRASIL, Edvan Alexandre De Oliveira, 88330-326 Balneário Camboriu - SC (BR); HUFF, Israel Fernandes, 88330-410 Balneário Camboriu - SC (BR); SANTIN, Marcos Ricardo, 88330-155 Balneário Camboriu - SC (BR); DUVOISIN, Charles Adriano, 88330155 Balneario Camboriu - SC (BR)
(74) Representative: V.O.
(86) International application number: PCT/BR2020/050272
(87) International publication number: WO 2021/012025

(57) **Abstract**

The present patent application relates to the ability of an electrical household appliance to electro pasteurize and electro sterilize food, clothes and similar utensils, through the synergy and harmonization of electric fields, magnetic fields and condensed electromagnetic waves, thus allowing modern homes to have a functional tool capable of supporting health more efficiently in their everyday environment, achieved through technology that also provides the ability to electro sterilize and electro pasteurize food, preserving the organoleptic properties thereof, thus making it possible, in addition to sanitizing food prior to ingestion, to preserve said food in a specific module, which can be an additional module on the common refrigerator, which will be applied to the field of methods for electro sterilizing and electro pasteurizing foods, utensils, linen and relevant objects relating to everyday domestic use.

## Description

### FIELD OF APPLICATION

The present invention patent application refers to an electrical household appliance and/or electro pasteurizer and electro sterilizer aggregating module for food and utensils, through harmonic synchrony of electric fields, magnetic fields and electromagnetic wave condensing system, belonging to the field of methods for electro sterilizing and electro pasteurizing of food, utensils, clothes and objects relating to everyday domestic use.

### BACKGROUND OF THE INVENTION

Currently, food poisoning and other diseases are caused by the lack of functional technologies capable of creating ways to sterilize objects employed in everyday use by people within their homes.

Thus, the author developed a technology capable of electro sterilizing and electro pasteurizing food and household utensils, via harmonic synchrony of electric fields, magnetic fields and electromagnetic waves condensed in equipment of functional use in contemporary homes.

Said equipment can be used commercially as a new electro sterilizer/electro pasteurizer of food and utensils, as well as a new aggregating module for refrigerators, the latter with the function of electro sterilizing/electro pasteurizing and preserving food.

It can also be used as an equipment capable of electro sterilizing/electrode contaminating clothes, as a "plus" in the operation of washing machines and can further be used in the most creative and ways and capable of being transformed into commercial equipment with the function of electro sterilizing and electrode contamination of any viable domestic inanimate device.

The great advantage of this technology is the speed of the sterilization process, as well as that it does not cause burning, as it does not use heat in the proposed system.

Thus, both food and utensils will be properly decontaminated in a quick and easy way, still preserving their organoleptic properties.

The sterilization processes by pulsed electric fields have been scientific knowledge for many years, as well as the sterilization processes by electromagnetic waves of ultraviolet, x-rays, and gamma rays; however, the Nobel Prize in Physics in 2018 evidenced the possibility of condensation of electromagnetic waves via physical means, that is, harmonized electric and magnetic fields means.

Thus, a sum and synergistic methodology of electric fields, electricmagnetic fields, and condensation of similar electromagnetic waves is proposed herein.

Providing interesting effects, where electro sterilization/electro pasteurization will be possible, in a time and cost-effective way and still preserving the organoleptic properties of the products thus processed.

### STATE OF THE ART

Therefore, in the state of the art there is a wide variety of solutions that provide for sterilization, pasteurization, and decontamination through the use of electrolysis, electric fields, magnetic fields, and trivial electromagnetic waves, but there is no technology that synergistically uses condensed and harmonized electromagnetic waves by electric fields and magnetic fields in mutual function.

Document BR 10 2018 068980-0 of 2018 describes a method and system for condensation of electromagnetic waves via modulated electric and magnetic fields and corresponding equipment, by Charles Adriano Duvoisin, describing the possibility of condensing electromagnetic waves via properly aligned electric and magnetic fields.

With this, it is possible to see the feasibility of new creations with the possibilities of electromagnetic waves condensation and also with a sterilizing expectation, since infrared waves, heat itself, radio frequencies, microwaves, ultraviolets, x-rays, and gamma rays, have the property and ability to sterilize under certain circumstances.

Thus, the invention herein promises a specific sterilizing innovation and is based on the passage and condensation of specific electromagnetic waves via pulsating electric and magnetic fields, thus completely differentiating itself from the aforementioned patent.

Document BR 10 2013 018666-0A2 of 2013 describes an equipment and continuous process for treating waste, food, and other materials via cold plasma sterilization, deep drying, compaction and packaging that comprises equipment
(1) deep drying, cold plasma sterilization and solid waste packaging intended to fulfill the functions of liquid withdrawal, plasma sterilization, and waste packaging.

The equipment (1) is intended for waste treatment processes including solid waste (R) being separated from liquid (L), drying, cold plasma sterilizing, and final waste packaging; elimination of microorganisms such as fungus and bacteria; the final waste can be stored for months without the characteristic bad smell and contamination by microorganisms such as fungus and bacteria; depending on the type of waste (R) entering the equipment (1), the final input can be used as fertilizer, input for burning in a boiler, input for energy generation, reused in pavements and other purposes.

The above described invention uses cold plasma to sterilize waste, but it is a static equipment and also does not use an electromagnetic wave condenser system. Said factors differ from the invention herein precisely because it is designed to be adapted to a plurality of home electrical household appliances to make them capable of carrying out electrolysis of food and utensils.

Patent document BR 10 2013 017403-3A2 of May 07, 2013 also published as US10143222B2 and WO2015000045A1, by the author himself, describes a compact device for electrolytic sterilization of food and utensils, especially for the electrical treatment of food and utensils using the electric current generated between a positive pole and a negative pole of an electrolysis system, all of them immersed in a fluid means contained within a container covered by a lid of said device, the container being the anode or positive pole and the lid by means of a cathode axis provided with a cathodic disk being the cathode or negative pole, and the electrolyte is water.

Thus, documents BR 10 2013 017403-3A2; US10143222B2 and WO2015000045A1, mentioned above, refer to a technology based on electric fields, derived from electrolysis, different from the invention herein, which deals with the interaction of condensed electromagnetic waves and the interaction between them, thus the distinctions between the state of the art presented and the object herein are clear, as we can reaffirm the prior art documents do not present the interaction of condensed electromagnetic waves, nor the interaction between them.

Numerous patents based on electromagnetic radiation with sterilizing actions could be cited as examples, as well as the use of microwave radiation, infrared/heat waves, Ultra-Violet waves, X-ray waves, gamma ray, etc. could be cited as well.

Thus, it is clear that electromagnetic waves already intrinsically possess the sterilizing property.

However, until today there has not been an invention that adds the electric fields action, more magnetic fields and condensed electromagnetic waves as electro sterilizing/electro pasteurizing synergistic agents being used as systems and methods for the manufacture of intelligent and special home appliances to the use of the salutary benefit of world homes.

### OBJECTIVES OF THE INVENTION

The objective is to create an electrical household appliance capable of electro sterilizing and electro pasteurizing food and household objects via synergistic sum of electric fields, magnetic fields, and condensed electromagnetic waves, thus creating a new platform of electric household appliances capable of assisting in the issue of domestic disinfection control.

### DESCRIPTION OF THE FIGURES

The attached drawings briefly show the electrical household appliance and/or electro pasteurizer and electro sterilizer aggregating module for food and utensils via harmonic synchrony of electric fields, magnetic fields, and electromagnetic wave condenser system, which together with the numerical references detailed below, is understood more easily, although this invention is capable of being implemented in many different constructive ways, always customized for each application, not shown in the drawings that will be described in detail herein and embodiments of said invention.
Figure 1 shows an example view of one of the ways of assembling a module to be applied to any electric household appliance, showing the alignment of electrodes and UV-C electromagnetic radiation emitting sources, between the upper and lower parts and identically between the right side relative to the left.
Figure 2 shows an example view of the module generating harmonic synchrony of electric and magnetic fields to form the electromagnetic wave condensing system.

### DETAILED DESCRIPTION OF THE INVENTION

This invention is based on an electrical household appliance capable of electro pasteurizing and electro sterilizing food and related utensils via harmonic synergism of Electric Fields, magnetic Fields and condensed electromagnetic waves.

The equipment is designed to be equipped with at least one electric and magnetic field generating system, which will be produced by electrodes that are positioned in parallel and in specific pairs.

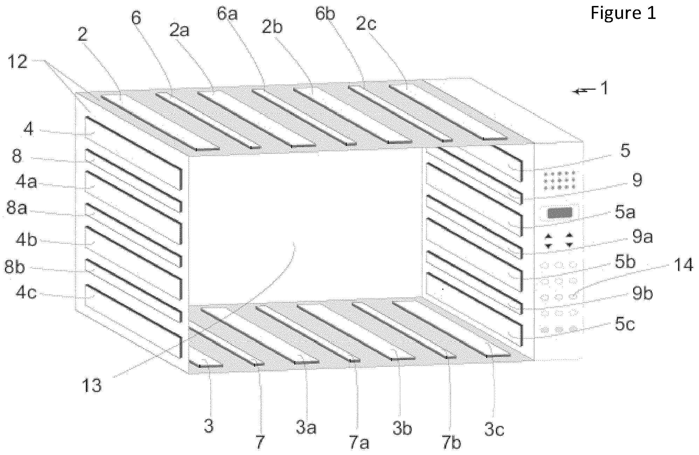

The module (1) to be incorporated into any electrical household appliance occurs by symmetrically aligning in parallel between the upper electrodes (2) and the lower electrodes (3) and so on, aligning in parallel the upper electrode (2a) with the lower electrode (3a); aligning in parallel the upper electrode (2b) with the lower electrode (3b); aligning in parallel the upper electrode (2c) with the lower electrode (3c); and so on, including more or less upper electrodes (2) and lower electrodes (3), according to the need defined in its use project (figure 1).

Similarly, on the sides, the positioning occurs within the module (1) of the symmetrical alignment in parallel between the right electrodes (5) with the left electrodes (4) and so on, aligning in parallel the right electrode (5a) with the left electrode (4a); aligning in parallel the right electrode (5b) with the left electrode (4b); aligning in parallel the right electrode (5c) with the left electrode (4c); and so on, including more or less right electrodes (5) and left electrodes (4), always with their pairs and in the same quantity so that they can be aligned with each other, the quantity being defined according to the project needs (figure 1).

The positioning in the module (1) to be incorporated into any electrical household appliance, occurs through the symmetrical alignment in parallel between the upper UV-C electromagnetic radiation emitting sources (6) lower UV-C electromagnetic radiation emitting sources (7) and so on, parallelly aligning the upper UV-C electromagnetic radiation emitting source (6a) with the lower UV-C electromagnetic radiation emitting source (7a); parallelly aligning the upper UV-C electromagnetic radiation emitting source (6b) with the upper UV-C electromagnetic radiation emitting source (7b); and so on, being able to contain more or less upper UV-C electromagnetic radiation emitting sources (6) and lower UV-C electromagnetic radiation emitting sources (7) in quantity to be defined according to the project needs (figure 1).

The same occurs on the sides with the symmetrical alignment in parallel between the right UV-C electromagnetic radiation emitting sources (9) with the left UV-C electromagnetic radiation emitting sources (8) and so on, aligning in parallel the right UV-C electromagnetic radiation emitting source (9a) with the left UV-C electromagnetic radiation emitting source (8a); aligning in parallel the right UV-C electromagnetic radiation emitting source (9b) with the left UV-C electromagnetic radiation emitting source (8b); and so on, including more or less right UV-C electromagnetic radiation emitting sources (9) that are aligned in parallel with the left UV-C electromagnetic radiation emitting sources (8) in an amount of use to be defined according to the project needs (figure 1).

The upper electrodes (2) are always interspersed by upper UV-C electromagnetic radiation emitting sources (6), and they will be respectively aligned in parallel with their respective lower electrodes (3) which are kept interspersed by the lower UV-C electromagnetic radiation emitting sources (7) and so on as more upper electrodes (2) and lower electrodes (3) are added, they identically increase for perfect assembly of the modules.

Between these upper electrodes (2), lower electrodes (3), right electrodes (5) and left electrodes (4) their respective upper UV-C electromagnetic radiation emitting sources (6), lower UV-C electromagnetic radiation emitting sources (7), right UV-C electromagnetic radiation emitting sources (9) and left UV-C electromagnetic radiation emitting sources (8) will be positioned, preferably UV-C and with the interaction of the fields produced by said electrodes, there will be a repulsive force and thus condensing the UV-C electromagnetic waves to more energetic waves and thus creating a new series of electromagnetic waves, such as UV-D, UV-F and UV-Z (10).

Thus, the condensed electromagnetic waves will also act in a sterilizing way and with the exclusive property of penetrating inside the materials to be sterilized, because with the condensation of the electromagnetic waves herein, they will become so small that they will easily have the penetration power in opaque materials, similarly to an X-ray type.

Thus, the juxtaposed electrodes system, such as the upper electrodes (2), lower electrodes (3), right electrodes (5) and left electrodes (4), will position their respective upper UV-C electromagnetic radiation emitting sources (6), lower UV-C electromagnetic radiation emitting sources (7), right UV-C electromagnetic radiation emitting sources (9) and left UV-C electromagnetic radiation emitting sources (8) successively, in a parallel and analogous way in pairs and interconnected to provide harmonic and synergistic electric and magnetic fields (figure 1).

The condensation of electromagnetic waves inside (13) the module (1) is directly proportional to the size of the generated electric and magnetic fields.

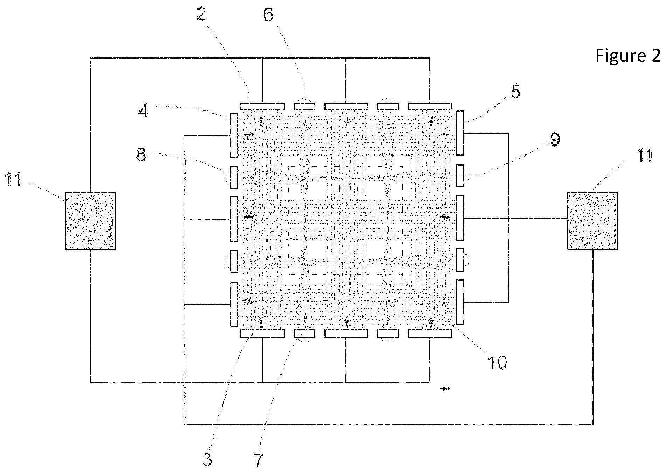

The electrodes will be connected and interconnected in a similar way and always respecting their pairs by high voltage sources (11), which can generate direct and alternating electric currents from 1 kV to 100 gV and also these high voltage sources (11) can generate pulsed frequencies and currents from 50 Hz to the maximum possible and feasible frequency to be produced, as it is known that the higher the frequency, the greater the sterilizing effect (figure 2).

The systems of ultraviolet/type C electromagnetic radiation emitting sources, interspersed between each electrode existing in the system so that the electric fields generated by the juxtaposed electrode pairs of this electric and magnetic field generator system force the compression on the size of the electromagnetic waves generated by ultraviolet/ type C electromagnetic radiation emitting sources, creating a greater energization on the electromagnetic waves, for said condensation of the electromagnetic waves.

The condensation of the electromagnetic waves emitted in UV-C, these will be condensed to UV-D or UV-F and so on, reaching up to UV-Z (10) indicated as an example, as well as the intercalated sources between the electrodes emit red light, which will become blue.

That is, the electromagnetic wave condenser system will occur with the synergism of the electric and magnetic fields generated with the magnetic force and also of the magnetic fields that will provide systems of polarization of the electromagnetic waves emitted by the intercalated emitters, between the corresponding electrodes and thus having in addition to a polarization of this same electromagnetic wave, also a condensation thereof, forcing waves the size of the UV-C emitted to become more energetic and transform into ultra violet waves with smaller sizes and higher energetic frequency, reaching UV-Z (10) indicated in the figures as an example.

The equipment housing is designed to suit the dimensions and propositions relevant to the possible and pertinent electrical household appliance models for electro pasteurization and electro sterilization.

The electrical isolation system between the electrodes, anti mismatch and short circuits is interconnected with the external housing, uniquely integrating the electrical isolation with opening parts such as the equipment doors.

The electrolytic means will be controlled with the use of aqueous media, specific gases, molten solids, thus becoming liquids, as well as uncontrolled electrolytic means will be used, such as air, public water, rivers, or any substance that serves as an electrolytic means.

This effect was cited in the book published in Germany in 2017 (Duvoisin et al, 2017)/Physical concepts of electrolysis, a new era of food safety. New Academic Editions/Germany, 2017 ISBN: 978-4-330-75839-1.

This system must work in isolation, that is, each electrode must be electrically isolated, thus avoiding undesirable short circuits.

There will also be a peripheral Faraday cage (12) indicated in (Figure 1) as an example, with the aim of isolating radio frequencies generated by it.

All equipment must have automatic safety systems in case of possible unexpected failures and burns.

The electrical supply of said equipment can be carried out by conventional electric currents and by common batteries.

All electrical household appliances must have one or more sterilizing/electro pasteurizing systems based on the harmonic action of electric fields and magnetic fields and condensed electromagnetic waves.

The electric and magnetic fields must be produced by electrodes arranged in parallel and juxtaposed (figure 1), and must also respect a synchrony of electrical pulses, which will provide the condensation of electromagnetic waves produced by a specific source, preferably the UV-C waves should be used, which, due to the intensity of the electric and magnetic fields generated, may be condensed into UV-Z waves (10), almost reaching energies similar to X-rays.

The effects of said radiations will be controlled by Faraday cages (12) and also by electrical insulation systems optimum for dimensioning the specific and desired electrical power.

The security system is composed of at least one metal grounded multilayer screen, with the function of the Faraday cage (12) for isolating radiofrequency waves and other possible undesirable discharge electrical currents.

This system can be executed in several ways, both with interleaved positioning of parallel and juxtaposed electrodes, respecting the independent or serial connection of sources generating specific high voltage electric currents (11) sized to generate harmonic electric fields.

These high voltage sources (11) can generate direct or alternating currents, as well as being pulsed with frequencies from 50 Hz to very high frequencies, as the electro pasteurizing and sterilizing effects are directly related to the electric fields generated and their attacks on frequencies, although there is already a reference of a minimum electric field per square centimeter recommended by the FDA, North American agency, which would be 20 kV/cm².

As for the frequencies, it can be seen that the higher the frequency, the greater the sterilizing effect, however the temperature can be increased, since some frequency bands provide resonance effects in some molecules and thus generating molecular friction by heating them.

For this reason, this system can be specifically modulated for each necessary case and thus providing ideal effects for each specific use case.

Electrical household appliances that can carry this electro sterilizer/electro pasteurizer technology can be of various shapes and sizes, from sterilizing electric pans, sterilizing electric ovens, refrigerators/freezers with special sterilizing and food preservation modules, washing machines with effective sterilization capacity, etc.

The advantages and necessity of the proposed innovation are directly related to the daily need to electro sterilize and electro pasteurize objects and foods in order to avoid cases of contamination, infections and diseases caused by the lack of preventive care and because there are no functional technologies that provide maintenance of sanitary conditions of various artifacts, or even food that will be precariously ingested and contaminated.

Thus, a revolutionary technology and the basis of the synergism of the known electric fields and magnetic fields with the action harmonization of condensed electromagnetic waves, called UV-Z, established by the 2018 Nobel Prize in Physics by the author, promises to be the great revolution for the issue of the invention of electro sterilizing/electro pasteurizing electrical household appliances, which will enable contemporary homes to decontaminate their food, clothes, utensils and everything else in a functional way, thus generating a safe and protected environment from routine infections and diseases.

There are several advantages of this technology, including speed, simplicity and time and cost-effectiveness, feasibility, compatibility and also for being able to sterilize preserving its constitutional organoleptic properties and thus preserving the fibers of clothes, utensils and especially the food nutritivity of said electro pasteurized and electro sterilized powders.

The electrical household appliances can be manufactured independently or aggregated by modules, that is, this electro sterilizer system can be linked to a common refrigerator, thus providing a type of food preserver without the need for cooling, because when an environment is sterilized, there will be no need for cooling and thus providing a cost-effective form of food conservation and also at room temperature, advantageously bringing to certain types of food, which can be consumed without being at a lower temperature than the environment and thus preserving even more their in natura state.

The equipment may have a control panel (14) designed to inform the powers, currents, safety alert, process time, temperature, possible burns from electromagnetic waves emitting sources and other components necessary for the perfect functioning of the electrical household appliance.

All the construction materials of this equipment may undergo evolutions with the futuristic technological development, that is, many electrically conductive materials, electrical insulators and other functions, have been developing in a surprising way with the advances of contemporary research and thus may also favor the invention herein.

The positioning of electrodes and UV-C electromagnetic radiation emitting sources may vary and only a lateral formation is used, to, for example, be used in the assembly in circular modules, which can be used in pans, washing machines, among other cylindrical equipment.

## Claims

1. Electrical household appliance and or electro pasteurizer and electro sterilizer aggregator module for food and utensils, through the harmonic synchronicity of electric fields, magnetic fields and electromagnetic wave condensing system, **characterized by** the ultra-violet/type C electromagnetic radiation emitting source systems, interspersed between each electrode in the system so that the electric fields generated by the juxtaposed electrodes pairs of this electric and magnetic field generator system force compression on the size of the electromagnetic waves generated by the ultra-violet/type C electromagnetic radiation emitting sources, creating a greater energization on the electromagnetic waves, for said electromagnetic waves condensation.

2. Electrical household appliance and or electro pasteurizer and electro sterilizer aggregator module for food and utensils, according to claim 1, **characterized by** the juxtaposed electrodes system, said upper electrodes (2), lower electrodes (3), right electrodes (5), and left electrodes (4) will position their respective upper UV-C electromagnetic radiation emitting sources (6), lower UV-C electromagnetic radiation emitting sources (7), right UV-C electromagnetic radiation emitting sources (9), and left UV-C electromagnetic radiation emitting sources (8) successively, in a parallel and analogous way in pairs and interconnected to provide harmonic and synergistic electric and magnetic fields.

3. Electrical household appliance and or electro pasteurizer and electro sterilizer aggregator module for food and utensils, according to claim 1, **characterized in that** module (1) is equipped with at least an electric and magnetic fields generating system, which will be produced by combining upper electrodes (2) laterally aligned and intercalated by an upper UV-C electromagnetic radiation emitting source (6) both aligned in parallel with the lower electrodes (3) laterally intercalated by lower UV-C electromagnetic radiation emitting source (7) always assembled in this way in specific pairs.

4. Electrical household appliance and or electro pasteurizer and electro sterilizer aggregator module for food and utensils, according to claim 1, **characterized in that** the module (1) incorporated into an electrical household appliance has the symmetrical alignment in parallel between the upper electrodes (2) and the lower electrodes (3) and so on, aligning in parallel the upper electrode (2a) with the lower electrode (3a); aligning in parallel the upper electrode (2b) with the lower electrode (3b); aligning in parallel the upper electrode (2c) with the lower electrode (3c); and so on, including more or less upper electrodes (2) aligned in parallel with the lower electrodes (3).

5. Electrical household appliance and or electro pasteurizer and electro sterilizer aggregator module for food and utensils, according to claim 1, **characterized in that** the module (1) incorporated into an electrical household appliance has on its sides the symmetrical alignment in parallel between the right electrodes (5) with the left electrodes (4) and so on, aligning in parallel the right electrode (5a) with the left electrode (4a); aligning in parallel the right electrode (5b) with the left electrode (4b); aligning in parallel the right electrode (5c) with the left electrode (4c); and so on, including more or less right electrodes (5) and left electrodes (4) always in the same amount to be aligned with each other.

6. Electrical household appliance and or electro pasteurizer and electro sterilizer aggregator module for food and utensils, according to claim 1, **characterized in that** the module (1) incorporated into an electrical household appliance has the symmetrical alignment in parallel between upper UV-C electromagnetic radiation emitting sources (6) with the lower UV-C electromagnetic radiation emitting source (7) and so on, aligning in parallel the upper UV-C electromagnetic radiation emitting source (6a) with the lower UV-C electromagnetic radiation emitting source (7a); aligning in parallel the upper UV-C electromagnetic radiation emitting source (6b) with the lower UV-C electromagnetic radiation emitting source (7b); and so on, including more or less upper UV-C electromagnetic radiation emitting sources (6) aligned in parallel with the lower UV-C electromagnetic radiation emitting sources (7).

7. Electrical household appliance and or electro pasteurizer and electro sterilizer aggregator module for food and utensils, according to claim 1, **characterized in that** the module (1) incorporated into an electrical household appliance has the symmetrical alignment in parallel between the right UV-C electromagnetic radiation emitting sources (9) with the left UV-C electromagnetic radiation emitting sources (8) and so on, aligning in parallel the right UV-C electromagnetic radiation emitting source (9a) with the left UV-C electromagnetic radiation emitting source (8a); aligning in parallel the right UV-C electromagnetic radiation emitting source (9b) with the left UV-C electromagnetic radiation emitting source (8b) and so on, including more or less right UV-C electromagnetic radiation emitting sources (9) aligned in parallel with the left UV-C electromagnetic radiation emitting sources (8).

8. Electrical household appliance and or electro pasteurizer and electro sterilizer aggregator module for food and utensils, according to claim 1, **characterized by** the condensation of electromagnetic waves emitted in UV-C, which will be condensed to UV-D or UV-F and so on, reaching up to UV-Z (10), and if the sources interspersed between the electrodes emit red light, they will become blue.

9. Electrical household appliance and or electro pasteurizer and electro sterilizer aggregator module for food and utensils, according to claim 1, **characterized in that** the equipment housing is designed to suit the dimensions and propositions relevant to the models of possible and relevant electric home appliances for electro pasteurization and electro sterilization.

10. Electrical household appliance and or electro pasteurizer and electro sterilizer aggregator module for food and utensils, according to claim 1 **characterized by** a system for high voltage sources (11) generating electric currents to feed the electrodes arranged in pairs and thus generating the electric and magnetic fields, the high voltage sources being continuous or alternating electric currents generating sources, operating at high voltages from 1kV to 100 gV.

11. Electrical household appliance and or electro pasteurizer and electro sterilizer aggregator module for food and utensils, according to claim 1, **characterized in that** the security system is composed of at least one metal grounded multilayer screen, with the Fareday cage (12) function of isolating radiofrequency waves and other possible undesirable discharge electrical currents.

12. Electrical household appliance and or electro pasteurizer and electro sterilizer aggregator module for food and utensils, according to claim 1, **characterized in that** the electrical isolation system between the electrodes, anti-mismatch and short circuit, is interconnected with the external housing, uniquely integrating the electrical insulation with opening parts such as the equipment doors.

13. Electrical household appliance and or electro pasteurizer and electro sterilizer aggregator module for food and utensils, according to claim 1, **characterized in that** the electromagnetic wave condenser system occurs with synergism of the electric and magnetic fields generated in a way with the magnetic force and also of the magnetic fields that will provide polarization systems of the electromagnetic waves emitted by the emitters interspersed between the corresponding electrodes and thus having, in addition to a polarization of said electromagnetic waves, also condensation thereof, forcing the emitted UV-C-sized waves to become more energetic and transform into smaller-sized, higher-frequency ultraviolet waves that can reach UV-Z.

14. Electrical household appliance and or electro pasteurizer and electro sterilizer aggregator module for food and utensils, according to claim 1, **characterized in that** the electrolytic means are controlled using aqueous means, specific gases, molten solids and thus becoming liquids, as well as using uncontrolled electrolytic means, such as air, public water, rivers or any substance that serves as an electrolytic mean.
